# EUROPEAN PATENT SPECIFICATION

(11) **EP 1 279 679 B1**
(45) Date of publication and mention of the grant of the patent: **08.07.2015**
(21) Application number: 01971447.6
(22) Date of filing: 11.04.2001
(51) Int. Cl.: C07K 14/44, G01N 33/68, A61K 39/008

(54) **COMPOSITION CONTAINING LEISHMANIA LIP2A**
VERBINDUNG MIT LEISHMANIA LIP2A
COMPOSITION CONTENANT LEISHMANIA LIP2A

(30) Priority: 17.04.2000 ES 200000999
(43) Date of publication of application: 29.01.2003
(73) Proprietor: Laboratorios Leti S.L. Sociedad Unipersonal, 08038 Barcelona (ES)
(72) Inventor: SOTO ALVAREZ, Manuel, Leganés E-28915 Madrid (ES); ALONSO BEDATE, Carlos, E-28015 Madrid (ES); REQUENA ROLANIA, José Maria, E-28700 Madrid (ES)
(74) Representative: Nederlandsch Octrooibureau
(86) International application number: PCT/ES2001/000147
(87) International publication number: WO 2001/081388

(56) References cited:
- WO-A-00/39298
- ES-A- 2 133 236
- SOTO M ET AL: "Genomic organization and expression of two independent gene arrays coding for two antigenic acidic ribosomal proteins of leishmania" THE JOURNAL OF BIOLOGICAL CHEMISTRY, vol. 268, no. 29, 15 October 1993 (1993-10-15), pages 21835-21843, XP002901845

## Description

This invention relates in general to polypeptides, proteins or nucleic acids that contain at least a portion of an acidic ribosomal protein from Leishmania infantum called Lip2a or a variation thereof, useful for modifying the immune response in immunised individuals or in cell groups. The invention relates specifically to compositions based on compounds from Leishmania infantum that are homologous to acidic ribosomal proteins, which are referred to -as Lip2a hereinafter, for stimulating immune responses and for use as vaccines or as therapeutic products.

The compositions of the invention are useful for promoting a humoral or cellular response in the individual who is inoculated with said compositions. Thus, the compositions of the invention can be used for treatment or prophylaxis of diseases. Specifically, among other applications, the compositions of the invention that use the Lip2a protein can be used as a adjuvant or as a subunit in a vaccine for the prevention of Leishmaniasis or treatment of other diseases.

### BACKGROUND OF THE INVENTION

Vaccines can induce protective immunity against an infection or disease by means of the generation of an appropriate immune response in an individual or patient who is suffering from such a disease. Currently, advances are being made in the development of vaccines against infectious agents and even against certain diseases, given that there are many appropriate techniques for the identification of antigens that have the potential to be used as agents able to induce specific responses to combat the most common infections and diseases such as viral, bacterial and protozoan infections, or even cancer.

In most cases, the immune response induced by the immunogen is weak and therefore the immune response generated, although directed against the antigen present in the pathogen, is not big enough to confer protection. In such cases, it is necessary to use an agent able to act as an adjuvant.

The adjuvants are substances that increase the specific response against a substance when they are injected A- before that substance B- in connection with that substance or Cat the same site as that substance or at different sites. In general, it can be said that the adjuvants contribute to an increase in the immune response in different ways: 1- They can help the antigen to be released slowly by/to the immune system; 2- by means of the stimulation and migration of antigen presenting cells to the injection site; 3- by means of the stimulation and proliferation of lymphocytes; and 4- by improving the dispersion of the antigen throughout the body of the patient.

This invention relates to a composition for stimulating an immune response in immunized individuals or in cell groups, characterized in that said composition consists of a saline solution that comprises Lip2a Leishmania protein represented by SEQ ID NO:2 or a polypeptide which differs there from only by conserved substitutions of the amino acids of SEQ ID NO:2 of which the amino acid groups that represent conserved changes are (1) ala, pro, gly, glu, asp, gin, asn, ser, thr; (2) cys, ser, tyr, thr; (3) val, ile, leu, met, ala, phe; (4) lys, arg, his; and (5) phe, tyr, trp, his, or comprises a nucleic acid encoding the Lip2a Leishmania protein as identified above, or being represented by SEQ ID NO:1. Preferably said composition is for use as vaccines or as therapeutic products.

The compositions of the invention are useful for promoting a humoral or cellular response in the individual who is inoculated with said compositions. Thus, the compositions of the invention can be used for treatment or prophylaxis of diseases. Specifically, among other applications, the compositions of the invention that use the Lip2a protein can be used as a adjuvant or as a subunit in a vaccine for the prevention of Leishmaniasis or treatment of other diseases.

### BACKGROUND OF THE INVENTION

Vaccines can induce protective immunity against an infection or disease by means of the generation of an appropriate immune response in an individual or patient who is suffering from such a disease. Currently, advances are being made in the development of vaccines against infectious agents and even against certain diseases, given that there are many appropriate techniques for the identification of antigens that have the potential to be used as agents able to induce specific responses to combat the most common infections and diseases such as viral, bacterial and protozoan infections, or even cancer.

In most cases, the immune response induced by the immunogen is weak and therefore the immune response generated, although directed against the antigen present in the pathogen, is not big enough to confer protection. In such cases, it is necessary to use an agent able to act as an adjuvant.

The adjuvants are substances that increase the specific response against a substance when they are injected A- before that substance B- in connection with that substance or C- at the same site as that substance or at different sites. I n general, it can be said that the adjuvants contribute to an increase in the immune response in different ways: 1- They can help the antigen to be released slowly by/to the immune system; 2-by means of the stimulation and migration of antigen presenting cells to the injection site; 3- by means of the stimulation and proliferation of lymphocytes; and 4- by improving the dispersion of the antigen throughout the body of the patient.

Oils, polymers, mineral salts and bacteria have been used and are currently used as adjuvants. Immunostimulatants are substances that induce a general or temporal immune response when administered with the antigen or separately. Typical immunostimulants are, for example, the Freund adjuvant (AC/IF), BCG (an attenuated species of Mycobacterium tuberculosis) or a non-viable form of Corynebacterium parvum, among others. The adjuvants or the immunostimulants act to increase the specific immune response by a non-specific route.

A serious drawback with most of the most potent adjuvants in use u until present is their high toxicity. Given that the mechanism of action of the adjuvants can be very varied and it is a requirement for the immune response against the associated protein to be directed against one part or another of the immune system, it is of utmost importance to identify compounds that are able to act: A- with adjuvant character (increase in the immune response both of the humoral type at the immunoglobin level, and of the specific cellular-type cytokines, preferably CD4+ or CD8+ or CTLs). B- that they have low or no toxicity. C- that they are formed of a substance that is as pure as possible, preferably a protein that although It has immunogenic character does not lead to problems of autoimmunity and a potential shadow effect on the immunogen with which it is administered.

The patent ES 2133236 relates to a chimeric gene comprising Lip2a. The resulting chimeric protein is used for the detection of leishmaniasis. Furthermore, the Lip2a protein has been explored as antigenic tester (Manuel Soto et al., The Journal of Biological Chemistry, Vol 268, No.29, p. 21835-21843, 1993), but no immunogenic activity of the protein with or without an adjuvant is reported.

The present invention describes for the first time the immunogenic activity of the Lip2a protein without the use of adjuvant.

### SUMMARY OF THE INVENTION

The present invention relates to compositions for stimulating an immune response in immunized individuals or in cell groups, characterized in that said composition consists of a saline solution that comprises Lip2a Leishmania protein represented by SEQ ID NO:2 or a polypeptide which differs there from only by conserved substitutions of the amino acids of SEQ ID NO:2 of which the amino acid groups that represent conserved changes are (1) ala, pro, gly, glu, asp, gin, asn, ser, thr; (2) cys, ser, tyr, thr; (3) val, ile, leu, met, ala, phe; (4) lys, arg, his; and (5) phe, tyr, trp, his, or comprises a nucleic acid encoding the Lip2a Leishmania protein as identified above, or being represented by SEQ ID NO:1. The present invention also relates to the capacity of the Lip2a protein to induce a specific immune response of the Th1 type when administered to an individual and to stimulate a type Th1 response *in vivo* and/or in mononuclear cells of individuals immunised with said protein.

The present invention also provides a method for producing specific immune responses in mononuclear cell samples by means of the incubation of cells with the protein called Lip2a from non-immunised individuals.

An alternative according to the invention to the use of the protein Lip2a for producing a Th1-type response is the use of viral vectors or nucleic acids that contain the gene Lip2a and are able to direct the expression of the Lip2a protein in Individuals or transfected cells with the composition of nucleic acids from SEQ ID NO 1.

### DESCRIPTION OF THE INVENTION

The present invention relates in general to an increase in the immune response that may be of humoral type or mediated by cells in an individual immunised with Lip2a as defined in claim 1 or in a cellular culture with the same protein, both in cells from the immunised individual or in mononuclear cells from healthy individuals.

Within the context of the invention, it is stated that Lip2a as defined in claim 1 is an immunostimulating compound (an immunogen against which an immune response is induced) and whose action can be initiated by itself without the need for adjuvants. Generally, the immune response against an antigen can be initiated or increased by the administration to the immunised person of a protein or adjuvant. Antigens and immunostimulating agents are generally protein molecules that proceed from virus, bacteria, parasites and tumours. Immunostimulators are molecules that direct the immune system response in one direction or the other with respect to Th1 or Th2-type cytokines or that mediated by CD4+ or CD8+ cells.

In this sense, in the context of this invention, the capacity of Lip2a to be used for treatment in those diseases that require a systematic increase of interferon-γ can also be included. Within the context of this invention, it is understood also the initiation of or immunostimulation against an antigen from a virus, bacteria, infectious agent or tumoral antigen, by means of the administration of said antigen with the protein Lip2a or derivates thereof.

The Lip2a protein belongs to a group of small molecular weight molecules of acidic character that interact with the ribosome during translation. This is a process common to organisms of three kingdoms: eubacteria, arche-bacteria and eukaryotes. The function of these proteins is to facilitate the interaction between the ribosome and some soluble translation factors (Sanchez-Madrid and co-workers, 1979; Möller and co-workers, 1983). The acid proteins bind to the large subunit of the ribosome forming complexes of two dimers by means of the interaction with another protein that shares certain characteristics with these dimers. This protein is denominated ribosomal protein P0 in eukaryotes and L10 in bacteria and archebacteria. In organisms of the latter two of these kingdoms, the dimers of acid proteins are formed by the same polypeptide, although in bacteria one of the copies undergoes post-translational modifications of acetylation (Terhorst and co-workers, 1972). In eukaryotes, the acid proteins, also called P proteins as they are phosphorylated in their active form (Wool, 1979; Hasler and co-workers, 1991) divide into two groups, P1 and P2, depending on how similar the sequences are and the presence of domain characteristics of each one of them. Thus in eukaryotes, the complex formed from binding to the ribosome is composed of two dimers in turn composed of a polypeptide from each group. In most eukaryotes, there is only one copy of the genes coding for the acid proteins of each group, although in Saccharomyces cerevisiae (Newton and co-workers, 1990) and Schizosaccharomyces pombe (Beltrame and Bianchi, 1990), two different acidic proteins have been described within each group, each one of them coded by different genes.

The protein Lip2a from Leishman infantum is composed of 106 amino acids with a molecular weight of 10.57 kDa (see SEQ ID NO 2). The analysis of the sequence of amino acids deduced from the sequence of cDNA L22 indicates that the protein Lip2a possesses characteristics common to those of the acidic ribosomal proteins from group P2 of eukaryotes, such as the highly conserved carboxy-terminus sequences, a very central area rich in pralines and alanines, and an amino-terminus with a more variable sequence (Soto and co-workers, 1993).

The compositions in accordance with this invention comprise a polypeptide that stimulates a strong humoral response in animals immunised therewith, even in the absence of adjuvants and a type-Th1 immune response in splenocytes of individuals that have or have not been immunised with Lip2a. This inducing polypeptide may comprise all or part of the complete protein from Leishmania infantum called Lip2a or a total or partial homologous ribosomal protein from another organism that has similar stimulatory activity.

The polypeptides according to the present invention include variants of Lip2a as defined in claim 1 able to produce a Th1 response in immunised individuals or in mononuclear cells in culture given the similarity between the proteins of this group. Such variants Include different structural forms of the native protein both in the form of a salt and in acid form or induced by modification of aminoacids, for example, through glycosylation. The polypeptides Lip2a expressed in E. coli are not glycosylated while the same ones produced in yeasts or in mammal cells are identical to those produced in E. coli, but they may differ structurally and in their immunological capacity due to glycosylation. The glycosylation sites are Asn-N-Pro(oS er). N can be any amino acid except Pro.

The polypeptides according to this disclosure comprise sequences of amino acids that do not differ substantially from Lip2a, where it is understood by such a concept modifications of amino acids that are coded for by DNA sequences able to form hybrids with the DNA sequences of the native Lip2a gene in not very stringent conditions. If these variations in the sequence of amino acids do not differ from Lip2a, it can be easily shown by its capacity to stimulate splenocytes in a similar fashion or as native Lip2a does. In general, these sequences, which do not substantially differ from native Lip2a, are formed of conserved substitutions of amino acids or formed by small substitutions or modifications, whether they natural or achieved through directed mutagenesis. In general, the amino acid groups that represent conserved changes are (1) ala, pro, gly, glu, asp, gln, asn, ser, thr; (2) cys, ser, tyr, thr, (3) val, ile, leu, met, ala, phe; (4) lys, arg, his; and (5) phe, tyr, trp, his. Within the scope of this disclosure there are included polypeptide variants of Up2a generated by covalent binding or through the construction of fusion proteins with other chemical or biological groups such as proteins using chemical or biological procedures among which are included the methods of recombinant DNA, for example, for a better or easier purification, greater stability, or formation of complexes that contain several total or partial Lip2a proteins.

The protein Lip2a can be expressed in E. coli or other prokaryotic or eukaryotic cells after transformation of these cells with the genomic sequence of the Lip2a gene or a total or partial cDNA clone of Lip2a. The nucleotide sequence of DNA of the Lip2a gene and of amino acids of the coded protein is shown the attached SEQ ID NO 1 and NO 2. The Lip2a gene can be isolated from the DNA of Leishmania infantum or another species of Leishmania or another organism by means of identification of cDNA clones from a gene expression library that expresses proteins of the organism by means of highly reactive animal sera naturally infected with Leishmania infantum or by PCR techniques using specific oligonucleotides.

In order to obtain the Lip2a gene, a cDNA gene expression library of Leishmania infantum was searched with dog serum infected naturally with Leishmania. The analysis of the sequence of three of the clones isolated indicated the existence of homology in the sequence with coding genes for the acidic type P2 ribosomal proteins. While the genes showed a total horology of the sequence in the coding region, untranslated 3' regions were completely divergent. There are at least two Lip2a genes in the genome of Leishmania infantum. These genes can be found grouped in the genome of the XIX chromosome. Variants of the Lip2a protein can be isolated by searching other gene expression libraries of cDNA of other species of Leishmania.

The protein Lip2a can be purified from bacterial cell cultures that express the Lip2a gene. The protein can be purified from the extracts or from the supernatants depending on the expression vector used both as a protein by itself or as a fusion protein with another protein. The methods vary although affinity chromatography, whether with specific antibodies against Lip2a or particle resins adapted to the synthesised protein, by the presence of a certain target that allows its binding to the resin, is the preferred method. Specifically, the Lip2a gene has been subcloned In a variant of the expression vectors pMAL and of the expression vectors pQE31 and the synthesised Up2a protein has been purified bound to the MBP protein in maltose columns, or bound to a chain of histidine amino acid with the aim of identifying its production and purification in columns of NT Ni. Similarly, monoclonal or mono-specific polyclonal antibodies may be used for its purification. Partial polypeptides that form the protein Lip2a and which form part of this invention can be synthesised from the construction of partial clones of Lip2a, known by all those well versed In the techniques of molecular biology.

An alternative method to the presentation of Lip2a protein as defined in claim 1 to individuals to be immunised or to culture cells is the administration of the protein in the form of nucleic acids that code for Lip2a or a portion of the protein in the form of plasmids, virus, included in liposomes or bound to polymers, or another way of introducing the protein into target cells. The vectors most used for the administration of the gene as an immunogen in the form of DNA are the so-called pcDNA3 and pRc/CMV under the control of specific promoters mostly of eukaryotic type. It is extensively documented in the literature that this type of immunisation preferably induces a response of the Th1 type.

The protein Lip2a as defined in claim 1 can be administered in conjunction with the antigen or separately, at the same time or at different times (for example, one day or two days before administration of the appropriate antigen), at the same site or In different sites of the animal. In this sense, Lip2a as defined in claim 1 could be used to induce a humoral response or as an adjuvant in vaccine preparations with antigens heterologous to those of leishmania.

Given that the response to the Lip2a protein may vary from individual to individual depending on whether that individual is infected with Leishmania or not and has therefore been exposed to the Lip2a protein or not the administration of protein may have a different immunological effect. In general terms, the Lip2a protein or the derivative polypeptides as defined in claim 1 are administered in pharmacological formulations as a single substance in a saline solution or in combination with appropriate physiological carriers, including anionic and cationic liposomes or other agents such as BCG, Bordatella peruses, or DNA and oligonucleotides 3'purine - 5'pyrimidine. The carriers should be non-toxic substances. The literature on the use of adjuvants or carriers is extensive and the most appropriate should be chosen for the desired administration route. For example, for oral administration, a solid compound should be chosen such as cellulose or glucose. The immunisation may be performed with a preparation of Lip2a as separate or bound molecules and in combination with one or more antigens and even cytokines that may act as immunostimulators. The routes and frequency of administration may vary extensively. The most used routes are intramuscular or subcutaneous, intranasal or oral, with a frequency of 3 doses every 15 days. The dose can also vary widely depending on the state of the individual and the size thereof. The dose of the Lip2a protein as defined in claim 1 as with any other immunogen, has to be adjusted so that a significant quantity of interferon gamma or other cytokines are induced. Normally, the dose may vary between 10 micrograms and 100 micrograms per 100 grams of the host.

Another alternative method of administration of Lip2a and induction of an immune response may be the extraction of cells (preferably peripheral mononuclear cells) from an individual, stimulate them *in vitro* with the Lip2a protein in the presence or not of an antigen (specific to a disease or infection) to subsequently reintroduce them into the patient with the aim of interfering with the pathological process. In this context the Lip2a protein as defined in claim 1 its polypeptide variants could be used as adjuvant or immunostimulator of a response. These characteristics of the Lip2a protein suggest that it may have a role to play in generating a protective or therapeutic immune response in patients with diseases depending on the presence of interferon-γ or parasites, for example, leishmaniasis. In this sense, the present invention describes systems for increasing or immunostimulating the humoral or cellular response in individuals or isolated cells (macrophages, monocytes, B cells or dendritic cells) from individuals who have been immunised or not with the Lip2a protein. The capacity to stimulate the production of interferon gamma shows the potential that Lip2a has to be widely used in therapy in a broad range of applications that require the induction of a non-specific response to this cytokine (not necessarily leishmaniasis).

As will be shown in the figures, which are cited by way of example later, the Lip2a protein is able to induce a strong humoral response in Balb/c mice immunised with 5 micrograms of the protein rLip2a in saline buffer by intraperitoneal route and an administration schedule of two doses with a three week separation between them (21 days). The data showed that high titres of immunoglobulins G and M are produced. After the first dose, the response was mainly of IgM type leading to a maturing towards IgG after the second inoculation. It was observed that the isotype of IgG most abundant in rats immunised with the protein rLip2a corresponded to the immunoglobin IgG2a although IgG1 was also produced. It is concluded from these data that the immunisation with the Lip2a antigen generates a mixed Th1/Th2 response, although Th1 predominates.

Given that the nature of this protein is conserved with others from other organisms, the specificity of the humoral response was analysed generated after immunisation. The recognition of the sera from immunised rats was analysed against a collection of synthetic proteins that comprise Lip2a. It was observed that the peptides A4, A5 and A6 were recognised by the sera. The region of the protein contained in said peptides is specific for the acid proteins of the trypanosomatides, with no activity having been detected against the peptide A7, which contains the carboxyl terminal conserved from the protein. This specificity of response was confirmed when a "Western Blot analysis was performed of the reactivity of the sera against the acidic proteins of Leishmania, MBP-Lip2a and MBP-Lip2b, human MBP-P2 and MBP-P2 of T. cruzi. There was only recognition towards the acidic proteins of parasites, the reactivity being greater against MBP-Lip2A, and in no case were antibodies generated against acidic proteins of the upper eukaryotic organisms tested (conserved with human P2 used in the assay). Thus, it was confirmed that the immunogenic protein or the immunostimulatant did not induce problems of autoimmunity in the host. Moreover, one of the peptides recognised by the sera of immunised animals coincided with the peptide recognised by the sera of the dogs infected with viscerocutaneous leishmaniasis. This means that also in dogs, this region of the protein is immunogenic in the natural infection with Leishmania.

The protein Lip2a also induces proliferation of splenocytes obtained from immunised mice. The results obtained showed that there was a direct relationship between the concentration of antigen and the level of cellular proliferation induced. Surprisingly, the same results were obtained when the proliferation of splenocytes obtained from non-immunised mice was analysed. In order to directly implicate the protein in proliferation, eliminating the possibility that contaminating bacteria might be provoking said phenomenon, the inhibitory capacity of an anti-Lip2a antibody generated in rabbit at different dilutions on cellular proliferation was analysed. It was observed that while a serum dilution of 1/100 induced a reduction in proliferation both for cultures with Concanavalin A and for cultures with the Lip2a antigen, although it was always greater in the case of cultures induced to proliferate with Lip2a, at higher dilutions (1/500 and 1/1000), inhibition was only induced in cultures with this antigen. This fact showed that the proliferative induction was dictated by the Lip2a protein and not by a contaminant thereof. The study of the kinetics of proliferation and the levels of the protein in mice immunised with the Lip2a protein and mice not immunised indicated that the Lip2a protein is able to induce the same proliferation kinetics and reach the same levels regardless of whether the animals have been immunized or not. This indicates that there should be cells that are able to respond to the Lip2a even in non-stimulated animals suggesting that during their life the animals have been exposed to proteins that have structural characteristics or a sequence similar to Lip2a. Overall, it can be affirmed from the data obtained that the protein rLip2a has very potent immunogenic and mitogenic characteristics.

To characterise the type of response generated during the proliferations, the lymphokines INF-gamma and IL-4 were analysed that are produced in the assays of proliferation, both of splenocytes from control rats that had not been immunized and from immunised rats. The results showed that the proliferation provoked by the antigen generates a significant increase in the secretion of INF-gamma in splenocytes of immunised rats. Similarly, a slight increase was detected in the levels of IL-4 in the cultures of splenocytes that proliferated in the presence of the antigen with respect to those that proliferate in the medium with no added stimulus. These results indicate that the response generated by the antigen provokes a cellular proliferation that generates a predominantly Th1 production pattern of lymphocytes.

With the aim of determining the potential protective character of the Lip2a, the protein was administered and subsequently the Balb/c mice were infected with the parasite. It was seen that there was a delay in the appearance of the disease and a reduction in the skin-type lesions generated by infections with Leishmania major. a total or partial cDNA clone of Lip2a. The nucleotide sequence of DNA of the Lip2a gene and of amino acids of the coded protein is shown the attached SEQ ID NO 1 and NO 2. The Lip2a gene can be isolated from the DNA of Leishmania infantum or another species of Leishmania or another organism by means of identification of cDNA clones from a gene expression library that expresses proteins of the organism by means of highly reactive animal sera naturally infected with Leishmania infantum or by PCR techniques using specific oligonucleotides.

In order to obtain the Lip2a gene, a cDNA gene expression library of Leishmania infantum was searched with dog serum infected naturally with Leishmania. The analysis of the sequence of three of the clones isolated indicated the existence of homology in the sequence with coding genes for the acidic type P2 ribosomal proteins. While the genes showed a total homology of the sequence in the coding region, untranslated 3' regions were completely divergent. There are at least two Lip2a genes in the genome of Leishmania infantum. These genes can be found grouped in the genome of the XIX chromosome. Variants of the Lip2a protein can be isolated by searching other gene expression libraries of cDNA of other species of Leishmania.

The protein Lip2a can be purified from bacterial cell cultures that express the Lip2a gene. The protein can be purified from the extracts or from the supernatants depending on the expression vector used both as a protein by itself or as a fusion protein with another protein. The methods vary although affinity chromatography, whether with specific antibodies against Lip2a or particle resins adapted to the synthesised protein, by the presence of a certain target that allows its binding to the resin, is the preferred method. Specifically, the Lip2a gene has been subcloned in a variant of the expression vectors pMAL and of the expression vectors pQE31 and the synthesised Lip2a protein has been purified bound to the MBP protein in maltose columns, or bound to a chain of histidine amino acid with the aim of identifying its production and purification in columns of NT Ni. Similarly, monoclonal or mono-specific polyclonal antibodies may be used for its purification. Partial polypeptides that form the protein Lip2a and which form part of this invention can be synthesised from the construction of partial clones of Lip2a, known by all those well versed in the techniques of molecular biology.

An alternative method to the presentation of Lip2a protein to individuals to be immunised or to culture cells is the administration of the protein in the form of nucleic acids that code for Lip2a or a portion of the protein in the form of plasmids, virus, including in liposomes or bound to polymers, or another way of introducing the protein into target cells. The vectors most used for the administration of the gene as an immunogen in the form of DNA are the so-called pcDNA3 and pRc/CMV under the control of specific promoters mostly of eukaryotic type. It is extensively documented in the literature that this type of immunisation preferably induces a response of the Th1 type.

The protein Lip2a can be administered in conjunction with the antigen or separately, at the same time or at different times (for example, one day or two days before administration of the appropriate antigen), at the same site or in different sites of the animal. In this sense, Lip2a could be used to induce a humoral response or as an adjuvant in vaccine preparations with antigens heterologous to those of leishmania.

Given that the response to the Lip2a protein may vary from individual to individual depending on whether that individual is infected with Leishmania or not and has therefore been exposed to the Lip2a protein, the administration of protein may have a different immunological effect. In general terms, the Lip2a protein or the derivative polypeptides are administered in pharmacological formulations as a single substance in a saline solution or in combination with appropriate physiological carriers, including anionic and cationic liposomes or other agents such as BCG, Bordatella peruses, or DNA and oligonucleotides 3'purine - 5'pyrimidine. The carriers should be non-toxic substances. The literature on the use of adjuvants or carriers is extensive and the most appropriate should be chosen for the desired administration route. For example, for oral administration, a solid compound should be chosen such as cellulose or glucose. The immunisation may be performed with a preparation of Lip2a as separate or bound molecules and in combination with one or more antigens and even cytokines that may act as immunostimulators. The routes and frequency of administration may vary extensively. The most used routes are intramuscular or subcutaneous, intranasal or oral, with a frequency of 3 doses every 15 days. The dose can also vary widely depending on the state of the individual and the size thereof. The dose of the Lip2a protein, as with any other immunogen, has to be adjusted so that a significant quantity of interferon gamma or other cytokines are induced that it can potentially induce. Normally, the dose may vary between 10 micrograms and 100 micrograms per 100 grams of the host.

Another alternative method of administration of Lip2a and induction of an immune response may be the extraction of cells (preferably peripheral mononuclear cells) from an individual, stimulate them *in vitro* with the Lip2a protein in the presence or not of an antigen (specific to a disease or infection) to subsequently reintroduce them into the patient with the aim of interfering with the pathological process. In this context the Lip2a protein or its polypeptide variants could be used as adjuvant or immunostimulator of a response. These characteristics of the Lip2a protein suggest that it may have a role to play in generating a protective or therapeutic immune response in patients with diseases depending on the presence of interferon-γ or parasites, for example, leishmaniasis. In this sense, the present invention describes systems for increasing or immunostimulating the humoral or cellular response in individuals or isolated cells (macrophages, monocytes, B cells or dendritic cells) from individuals who have been immunised or not with the Lip2a protein. The capacity to stimulate the production of interferon gamma shows the potential that Lip2a has to be widely used in therapy in a broad range of applications that require the induction of a non-specific response to this cytokine (not necessarily leishmaniasis).

As will be shown in the figures, which are cited by way of example later, the Lip2a protein is able to induce a strong humoral response in Balb/c mice immunized with 5 micrograms of the protein rLip2a in saline buffer by intraperitoneal route and an administration schedule of two doses with a three week separation between them (21 days). The data showed that high titres of immunoglobulins G and M are produced. After the first dose, the response was mainly of IgM type leading to a maturing towards IgG after the second inoculation. It was observed that the isotype of IgG most abundant in rats immunised with the protein rLip2a corresponded to the immunoglobin IgG2a although IgG1 was also produced. It is concluded from these data that the immunisation with the Lip2a antigen generates a mixed Th1/Th2 response, although Th1 predominates.

Given that the nature of this protein is conserved with others from other organisms, the specificity of the humoral response was analysed generated after immunisation. The recognition of the sera from immunised rats was analysed against a collection of synthetic proteins that comprise Lip2a. It was observed that the peptides A4, A5 and A6 were recognised by the sera. The region of the protein contained in said peptides is specific for the acid proteins of the trypanosomatides, with no activity having been detected against the peptide A7, which contains the carboxyl terminal conserved from the protein. This specificity of response was confirmed when a "Western Blot" analysis was performed of the reactivity of the sera against the acidic proteins of Leishmania, MBP-Lip2a and MBP-Lip2b, human MBP-P2 and MBP-P2 of T. cruzi. There was only recognition towards the acidic proteins of parasites, the reactivity being greater against MBP-Lip2A, and in no case were antibodies generated against acidic proteins of the upper eukaryotic organisms tested (conserved with human P2 used in the assay). Thus, it was confirmed that the immunogenic protein or the immunostimulatant did not induce problems of autoimmunity in the host. Moreover, one of the peptides recognised by the sera of immunised animals coincided with the peptide recognised by the sera of the dogs infected with viscerocutaneous leishmaniasis. This means that also in dogs, this region of the protein is immunogenic in the natural infection with Leishmania.

The protein Lip2a also induces proliferation of splenocytes obtained from immunised mice. The results obtained showed that there was a direct relationship between the concentration of antigen and the level of cellular proliferation induced. Surprisingly, the same results were obtained when the proliferation of splenocytes obtained from non-immunised mice was analysed. In order to directly implicate the protein in proliferation, eliminating the possibility that contaminating bacteria might be provoking said phenomenon, the inhibitory capacity of an anti-Lip2a antibody generated in rabbit at different dilutions on cellular proliferation was analysed. It was observed that while a serum dilution of 1/100 induced a reduction in proliferation both for cultures with Concanavalin A and for cultures with the Lip2a antigen, although it was always greater in the case of cultures induced to proliferate with Lip2a, at higher dilutions (1/500 and 1/1000), inhibition was only induced in cultures with this antigen. This fact showed that the proliferative induction was dictated by the Lip2a protein and not by a contaminant thereof. The study of the kinetics of proliferation and the levels of the protein in mice immunised with the Lip2a protein and mice not immunized indicated that the Lip2a protein is able to induce the same proliferation kinetics and reach the same levels regardless of whether the animals have been immunised or not. This indicates that there should be cells that are able to respond to the Lip2a even in 25 non-stimulated animals suggesting that during their life the animals have been exposed to proteins that have structural characteristics or a sequence similar to Lip2a. Overall, it can be affirmed from the data obtained that the protein rLip2a has very potent immunogenic and mitogenic characteristics.

To characterise the type of response generated during the proliferations, the lymphokines INF-gamma and IL-4 were analysed that are produced in the assays of proliferation, both of splenocytes from control rats that had not been immunised and from immunised rats. The results showed that the proliferation provoked by the antigen generates a significant increase in the secretion of INF-gamma in splenocytes of immunised rats. Similarly, a slight increase was detected in the levels of IL-4 in the cultures of splenocytes that proliferated in the presence of the antigen with respect to those that proliferate in the medium with no added stimulus. These results indicate that the response generated by the antigen provokes a cellular proliferation that generates a predominantly Th1 production pattern of lymphocytes.

With the aim of determining the potential protective character of the Lip2a, the protein was administered and subsequently the Balb/c mice were infected with the parasite. It was seen that there was a delay in the appearance of the disease and a reduction in the skin-type lesions generated by infections with Leishmania major.

### DESCRIPTION OF THE FIGURES

The invention will now be described with reference to the attached figures, in which:
Figure 1 describes the system used for isolating the Lip2a gene from Leishmania infantum and in whose genome there are two copies of this gene.
Figure 2 shows the expression (RNA) of the gene of the Lip2a protein in Leishman infantum.
Figure 3 shows the expression of the Lip2a protein in bacterial cultures of E. coli transformed with the pMal expression vector that contains the fusion protein MBP-Lip2a and its purification and recognition of the Lip2a protein by sera from animals naturally infected with Leishmania.
Figure 4 shows the specificity of the immune response to Lip2a of sera of animals infected naturally by Leishmania.
Figure 5 shows the localisation within the protein of the determining antigens recognised by the sera of the animals infected naturally by Leishmania infantum.
Figure 6 shows that the Lip2a protein is homologous to the acidic ribosomal proteins P2 of other eukaryotic organisms.
Figure 7 shows that the Lip2a protein is able to induce a humoral response after inoculation of mice.
Figure 8 shows the specificity of the response to peptides of Lip2a from sera of Balb/c animals inoculated with Lip2a and the specificity of the sera of mice immunised against proteins of the P2 family.
Figure 9 shows that the protein is able to induce a proliferative response in splenocytes of animals immunised and not immunised.
Figure 10 shows that the Lip2a protein confers a certain degree of protection and delays the appearance of symptoms of infection by Leishmania major.

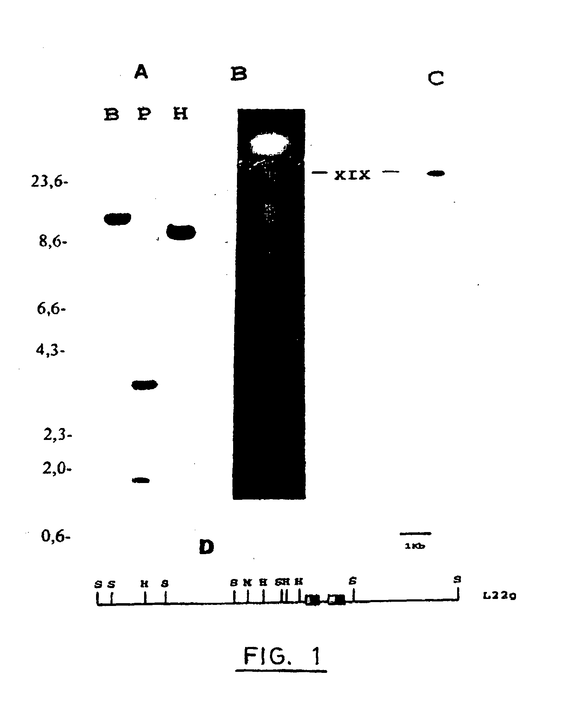

With reference to Figure 1, the cDNA expression library of Leishmania infantum was searched with the serum of a dog infected naturally with Leishmania. To characterise the sequences of DNA contained in the isolated phages, the cDNA inserts contained therein were cloned and sequenced in the plasmid pUC18. The analysis of the sequence of three of the isolated clones (L 21, L 22 and L 23) indicated the existence of homology of the sequence with the genes coding for the eukaryotic acidic ribosomal proteins of the P2 type. The cDNAs L21 and L22 had an identical sequence throughout their length, with L22 (454 nt) being the greatest in both cases. When the sequence of clone L22 was compared with that of clone L31, noteworthy differences were observed: While the homology of the sequence from the clones L22 and L31 was complete in the coding region, the untranslated regions 3' were absolutely divergent. The protein was denominated Lip2a.

In order to study the organisation of the genes Lip2a, Southern Blot analyses were performed. In section A of **Figure 1** there is shown the result of the hybridisation of the insert of clone L22 against filters that contained DNA from L. infantum digested with different restriction enzymes (B = Bam H I; H = Hind III; P = Pst I). The pattern obtained from using L 22 as a probe indicates that there are at least two Lip2a genes, as two bands of hybridisation appear in the Pst I lane, an enzyme that does not present a cut sequence in L 22 cDNA. These genes are found grouped in the genome, as only one hybridisation band was obtained in the DNA lanes digested with the rest of the restriction enzymes used. In fact, and as can be deduced from the pattern of hybridisation obtained after hybridising the insert of clone L 22 to filters that contained Leishmania chromosomes separated in pulsed field, the Lip2a genes are localised on a single chromosome (XIX chromosomal band) (sections B-C).

In order to define in detail the sequence and organisation of the genes coding for the acidic protein Lip2a from Leishmania, the insert of clone L22 was used to search a genomic gene library of this parasite, constructed in the replacement vector EMBL-3. A positive phage was isolated, denominated L22g. The restriction map of the phage (D), as well as the data for the sequence that are detailed afterwards, showed that there were two genes coding for the protein Lip2a, formed by two units organised in tandem. The sequence of the fragment Sal I of 2.8 Kb of the phage L22g that contained the genes responsible for cDNA L22 are shown in SEQ ID NO 1 attached. The coding zone of the two genes did not show differences except two transitions that do not lead to changes in the amino acid sequence of the coded protein; on the contrary, zones 5' and 3' that are not translated are completely different.

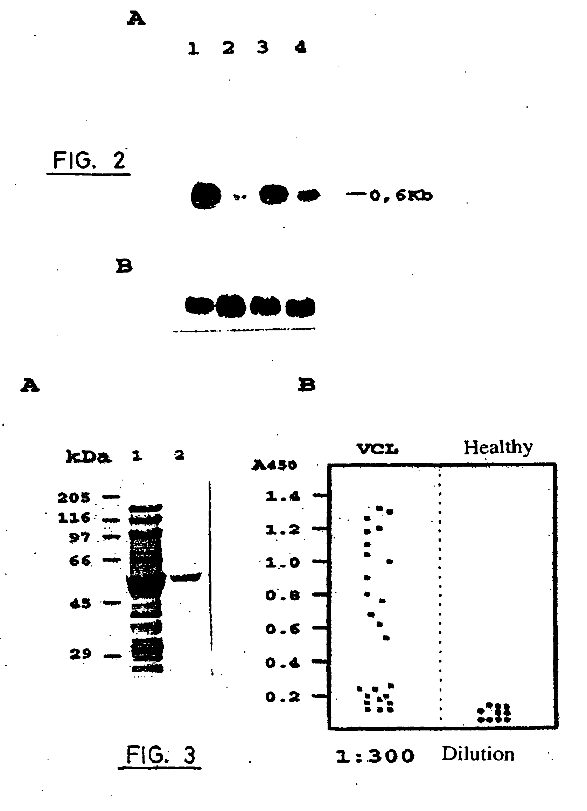

Hybridisation studies on RNA of Leishmania in filters, using the insert of clone L22 as a probe, showed that the size of the transcripts of the Lip2a genes was 0.6 Kb (see **Figure 2-A**). These messenger RNAs are polydenylated. As is shown in **Figure 2** (panel A, lanes 1 and 2), there were changes in the intensity of the bands of hybridisation obtained, according to whether RNA was obtained from cultures of promastigotes growing logarithmic or stationary phase. From this it is deduced that the expression of the genes of the acid protein Lip2a from Leishmania is greater in logarithmic phase. This type of regulation in the expression of genes from the acidic proteins has also been described in yeasts (Newton and co-workers, 1990, Saenz-Robles, 1990). The treatment of thermal shock, induced on incubating cultures of promastigotes in logarithmic phase for 2 hours at 42° C also led to a reduction in the levels of transcripts of the acidic proteins of the parasite (panel A, lane 4). On the other hand, and as is shown in lane 2 (panel A), the hybridisation obtained in the lane of axenic amastigotes was up to 6 times lower than in the lane of RNA extracted from promastigotes. The data seem to indicate that the mode of expression of the Lip2a genes of Leishmania is linked to the cellular metabolic state (Soto and co-workers, 1993).

According to **Figure 3****,** in panel A (line 1), the expression of the Lip2a protein is shown in a bacterial culture of E. coli transformed with the expression vector pMAI-cRI that contains the Lip2a gene and its purification (line 2). The molecular weight of the purified protein corresponds to a fusion protein MBP-Lip2a. MBP means the maltose binding protein of E. coli that is used to form the fusion protein with Lip2a. Panel B shows the recognition of the Lip2a protein by a collection of sera from animals with leishmania infected naturally.

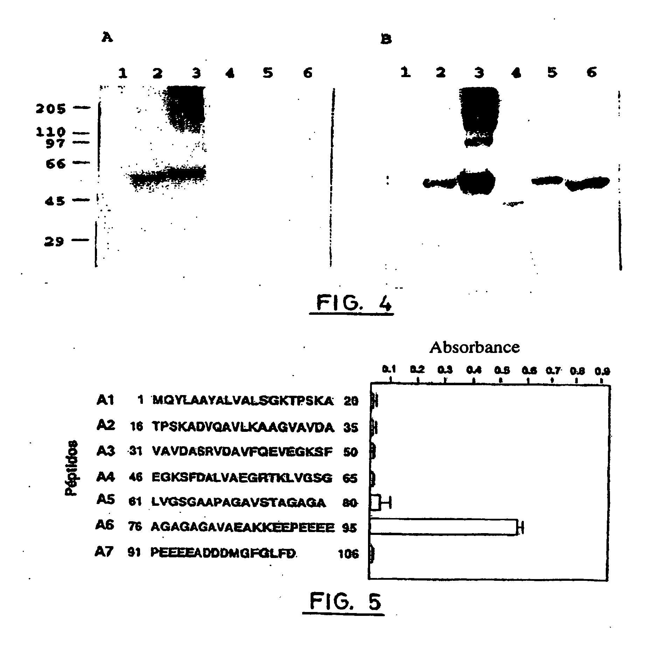

In accordance with **Figure 4****,** the specificity of the humoral response was analysed generated against Lip2a by means of measurement of the reactivity of sera obtained from dogs infected with Leishmania against homologous proteins from other species. In the case of the Lip2a acidic protein, the response of positive canine sera was analysed against another acidic protein from Leishmania, LiP2b, the human acidic proteins P1 and P2 and P2 from T. cruzi, all expressed as fusion proteins after cloning in the pMAL-cRI vector. In panel A of the figure, there is shown the result of the incubation of a set of sera obtained from dogs infected with Leishmania against the acidic proteins described previously. Only the fusion proteins MBP-Lip2a and MBP-Lip2b (lines 2 and 3) were recognised, indicating that the antibodies produced against the acidic proteins during the process of leishmaniasis are directed specifically against the acidic proteins of Leishmania. In panel B of the figure, it is shown that the serum from a patient with systemic lupus erythematosus reacted to the expressed proteins from the three species. This result indicated that the acidic proteins from Leishmania contain the antigenic determinant against which the humoral response originates during the Chagas disease and in human autoimmune processes, localised at the carboxy terminus of the protein. However, the lack of reactivity against the human acidic proteins or those of T. cruzi in dog sera infected with Leishmania shows that there are no antibodies directed against the C-terminus of these proteins in sera from animals with leishmaniasis.

In order to localise the antigenic determinants of Lip2a, peptides were synthesised that overlap by 5 amino acids from the sequence of amino acids of the Lip2a protein. **Figure 5** shows the sequence of peptides and the values of optical density obtained when these peptides are analysed in FAST-ELISA assays against sera of infected dogs. Only positive values were obtained against the peptide A6. The lack of reactivity obtained after testing the sera of animals infected with Leishmania against peptide A-7, which contains the conserved C-terminus sequence in acidic proteins of eukaryotes, confirmed that this zone does not generate any humoral response during infection with the parasite.

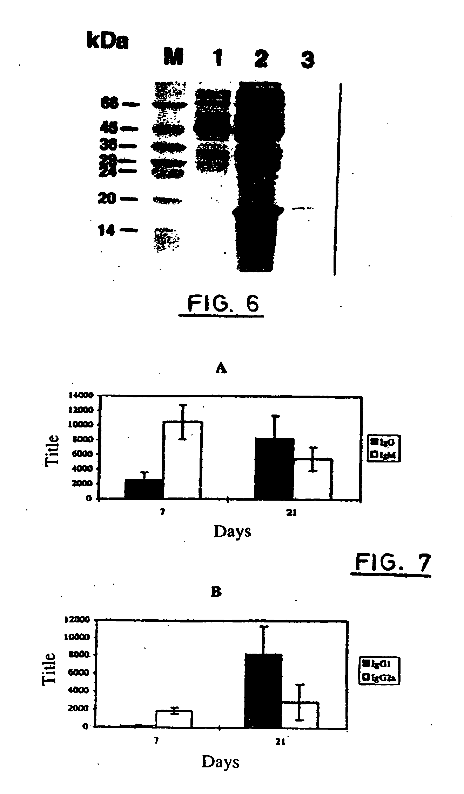

In order to be able to analyse the potential immunogenic or immunostimulating role of Lip2a from Leishmania an insert of the clone L22 was cloned in the eukaryotic expression vector pQE-31. The induction of bacterial cultures transformed with the recombinant plasma pQE-Lip2a produced the overexpression of a protein that corresponded to Lip2a expressed in recombinant fashion, denominated rLip2a. **Figure 6** shows the result of the expression and subsequent purification of the protein from 500 ml of culture, induced by IPTG, by means of affinity chromatography in NT-Ni columns. The bacteria were solubilised in 8 M urea and sonicated. Molecular weight marker proteins (lane M). E. coli proteins without transformation by the expression vector pQE-31 (lane 1). Proteins of E. coli transformed with the expression vector pQE-31 (lane 2). The protein was purified from the soluble fraction (lane 3).

The protein expressed in the vector pQE-31 includes the Lip2a protein and those amino acids that were included during the cloning process. The Lip2a protein is composed of 106 amino acids and lies between amino acid 39 and 144 (SEQ ID NO 3) and has a molecular weight of 10.57 kDa. (See SEQ ID NO 3 hereinafter).

The analysis of the sequence of amino acids deduced from the cDNA L22 sequence indicated that the Lip2a protein has characteristics in common with acidic ribosomal proteins of the P2 group of eukaryotes. Comparison of the Lip2a protein from Leishmania deduced from the sequence of nucleotides shows an identity of 89.62% with its homologue from Leishmania donovani (RLA2 LEIDO), an identity of 81.13% with its homologue from Leishmania brasiliensis (RLA2 LEIBRE), of 61.46% with its homologue from Trypanosoma cruzi (RLA2 TRYCR), of 43.47% with its homologue from humans (RLA2 HUMAN) and 51.87% with Lip2b from Leishmania infantum (RLA2 LEIIN). The greater homology is shown at the amino and carboxy termini. The protein has a very flexible central zone rich in prolines and anilines. In the sequence figure (see later) there are shown with a vertical bar the amino acids that are identical in the four proteins Lip2a, Lip2b from Leishmania, TcP2 homologue from Trypanosoma cruzi (RLA2 TRYCR) and HuP2 human homologue (RLA2 HUMAN).

A total of 12 BALB/c mice were inoculated with 5 micrograms of the rLip2a protein soluble in saline buffer. The route of inoculation was intraperitoneal, and the immunisation schedule was of two doses separated between them by three weeks (21 days). Blood was taken from mice on days 0, 7 and 28 and the respective sera were analysed. **Figure 7** (panel A) shows the titres of immunoglobulins G and M present in the serum of mice on days 7 and 28, indicating that after the first dose the response was mainly IgM, with a maturing towards IgG after the second inoculation. Given that the cytokines related with a Th1 response produce a response mainly of IgG2a and the cytokines secreted by the overpopulations of Th lymphocyte helpers stimulate an IgG1 response, the titres of the most abundant subtypes of IgG generated after the two doses of antigen were analysed. **Figure 7B** shows that the isotype of IgG most abundant in mice immunised with the rLip2a protein corresponded to the IgG2 immunoglobulin after the first dose and that the Th2 increases after the second dose. It is concluded that immunisation of this antigen generates a mixed Th1/Th2 response, although the response is mainly Th1 in the first weeks of immunisation. The proteins used in this study were purified from endotoxins by means of polymixin columns.

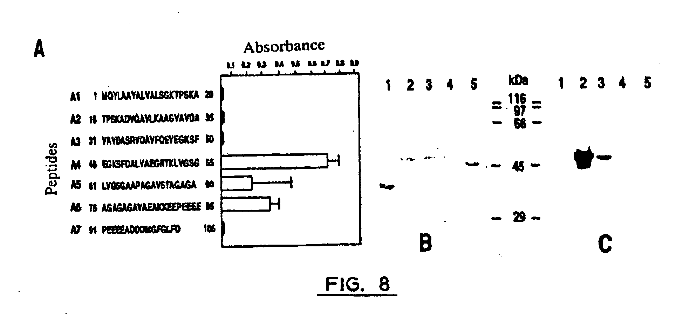

Taking into account the conserved nature of this protein, a study was necessary of the specificity of the humoral response generated after the immunisation. With this aim, the recognition of the sera from immunised mice was analysed (sera obtained on day 28) against synthetic peptides. **Figure 8-A** illustrates the results obtained, showing the recognition of the sera from immunised mice towards three of the peptides, A4-A6. The region of protein contained in said peptides is specific for the acidic proteins of the trypanosamatides, with no reactivity being detected against the peptide A7, which contains the conserved carboxyl terminus of the protein. This specificity of response was confirmed when the "Western Blot" analysis was conducted to analyse the reactivity of the sera against acidic proteins from Leishmania, MBP-Lip2a and MBP-Lip2b, human MBP-P2 and MBP-P" from T. cruzi. Recognition was only obtained towards the acidic proteins from parasites, with the reactivity being highest when generated against MBP-Lipsa (panel B, line 2) and in no case were antibodies generated against the acid proteins from superior eukaryotes (conserved with the human P2 used in the assay). Finally, it can be mentioned that one of the peptides recognised coincided with the peptide recognised by the serum of the dogs affected by viscerocutaneous leishmaniasis.

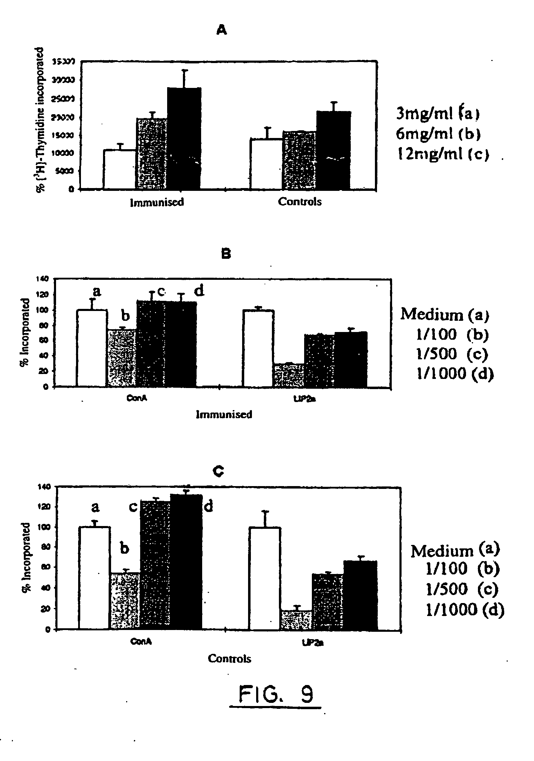

The spleens were extracted from mice that have been immunised two weeks after the last immunisation and its proliferation was analysed against different concentrations of antigen. The results tabulated in **Figure 9-A** show the values (in cpm) of thymidine tritiade incorporated by the splenocytes of mice immunised after 96 hours. The values obtained showed that there was a direct relation between the concentration of antigen and the cellular proliferation. Surprisingly, the same results were obtained when the proliferation of splenocytes was analysed that were obtained from non-immunised mice. This result indicates that the Lip2a protein has very potent immunogenic characteristics.

In order to directly implicate the proliferation protein, eliminating the possibility that bacterial contaminants could be provoking said phenomenon, the effect of the presence of an anti-Lip2a antibody generated in rabbit was analysed. Panels B and C of Figure 9 show the effect on proliferation of different dilutions of antibody. Thus, while the 1/100 dilution provoked a reduction in the proliferation induced both by a mitogen (concanavalin A, -ConA) and by the antigen, although it was always greater in the latter case, higher dilutions (1/500 and 1/1000) only led to a reduction in the proliferation induced by the antigen.

To characterise the type of response generated, the lymphokines INF-gamma and IL-4 produced in the proliferation assays were analysed, both for the splenocytes from control mice and from immunised mice. In the table given below, the results obtained are shown. These indicate that the proliferation provoked by the antigen generates a significant increase in the secretion of INF-gamma, especially in splenocytes of immunised mice. Similarly, a slight increase was detected in the levels of IL-4 in the cultures of splenocytes that proliferated in the presence of antigen with respect to those that proliferated in the medium with no added stimulus. These results indicate that the response generated by the antigen provokes a cellular proliferation that leads to the production of a pattern of lymphokines in which the Th1 type predominates. These results suggest that the Lip2a protein can be used as an adjuvant or as a subunit in a vaccine and thus could be relevant for treatment and/or prevention of diseases present in animals and humans.

| | | IL-4 (pg/ml) | INF-gamma (ng/ml) |
|---|---|---|---|
| Immunised | ConA | 99 | 8.4 |
| | Lip2a | 80 | 5.8 |
| | Medium | 40 | 0.5 |
| Controls | ConA | 98 | 6.1 |
| | Lip2a | 61 | 1.2 |
| | Medium | 13 | 0.4 |

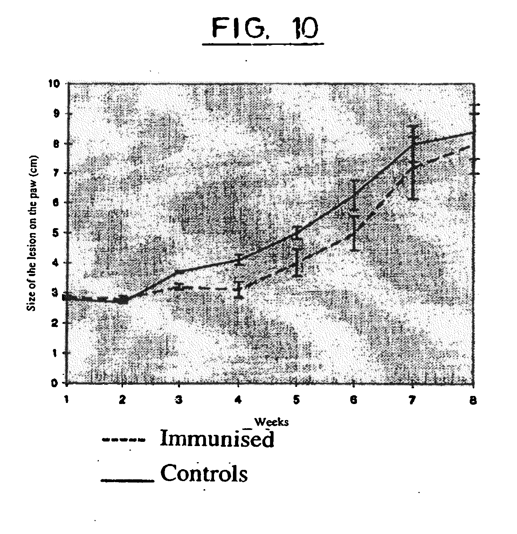

Three doses of 5 micrograms of Lip2a were administered in PBS at intervals of 15 days to a group of four BALB/c mice (5 micrograms per mouse). One week after the last dose, they were infected with 5 x 10⁴ promastigotes from an infective strain of L. mayor in the plantar pad of the right paw of both immunised mice and those corresponding to controls (who received only PBS). The size of the lesion on the paw was measured weekly, obtaining the results that are shown in Figure 10. The results obtained indicated that the immunisation of the protein Lip2a led to a delay in the appearance of lesions of two weeks, with the size of the lesion not increasing until week 5 in the immunised mice, while in the non-immunised controls, the lesions appear after the third weeks. In addition, the swelling provoked by the infection in immunised mice is less that in the controls, until it becomes the same from week 8 onwards.
SEQ ID NO:1 from the fragment Sal 1 - Sal 1 L22g. The nucleotide séquense of the clones of cDNA L22 and L31 is shown in boldface. 
SEQ ID NO 1

1 70
gtcgacggcg acatggctag agcagtttgt gcagctcccg ctgagcgaca agaaacacga tctgccgcgt

71 140
cgccacggag gtgtttctcc atgcctttgt ccagtgcatc aatgagtccg ggatctttgt cgcaccacca

141 210
gcaaaagagg agcagtgcct actcgcggcc ttaggaacgg caatgcgacc ttttgcacag taccccgagg

211 280
agacgatcgc ccaggcaaat gcgtttctgc atcaaggagg gcttccgcat gtcccgttcg cagctgaggc

281 350
ggtggagcag caggttatga atctccagcg gctgcattaa tcgccgcctg ccagacaccg ggaggtcct

351 420
ctgtttctgt ttttgcgtgt tgcgtctttc tctttatgtt tgctcctttg tgtctgtcgg ttaagagctc

421 490
ctcccttgcc cagaaaacag gagtaaccga gtacgccgca gcgcctgcgc cacacgttgt ccatggaacc

491 560
cctcccctcc tcgctcctcc cttctccact ccctccttct gggtgctgca tgtgtgtgtg catgtgtgtg

561 630
taactttgcc tcggtgtggc tggcacgctg cgccccctcc cccccccccc ccaaaaaaaa aaaacagcat

631 700
catcagtggg ctgacctgga tacatctcct cctctccttg tgttccccat cccctcttcg ctcttcctct

701 770
atgcacctcg cccactgcgc gcatcacgca cgcatcatcg cggctacgga acacgcgacc cccaccccac

771 840
ataggttttt tcaacgagaa atgcagtacc ttgccgcgta cgccctcgtg gcgctgtctg gcaagacgcc
[L22
841 910
gtcgaaggcg gacgttcagg ctgtcctgaa ggccgccggc gttgccgtgg atgcctcccg cgtggatgcc

911 980
gtcttccagg aggtggaggg caagagcttc gatgcgctgg tggccgaggg ccgcacgaag ctggtgggct

981 1050
ctggctctgc cgctcctgct ggcgctgtct ccactgctgg tgccggcgct ggcgcggtgg ccgaggcgaa

1051 1120
gaaggaggag cccgaggagg aggaggccga tgatgacatg ggcttcggtc tctttgacta agcagccccg

1121 1190
cactgcgctg caggcgcctc tgccgaagat tctcacgcgg gcctgctctc attgttgtga tgcatcgttt
 
1191                                                                    1260
ctttctttgc ttgtgacttc ggttcgtctt ttgatttcga gtggaaagac tctgcaaatc gaacaacccg
                                          L22]
1261                                                                    1330
tgcgagatga gctgggagcg taggcgaggt ggctgctcgc gaggctgtaa cgaaaaaaaa aagacagcag

1331                                                                    1400
cggcgccctc ggcacaaaca cagcgagccc tcccctcccc cgcttcgtcc ctcctcgaga agagagagac

1401                                                                    1470
aaagaatctc cacagacgct gtacgagagg caccggcctc gtcatcgaga gaagcaaccg cgctttcgtg

1471                                                                    1540
ccgtgacccg ctgaccttcg ataaccgaga gagggtgtct tctcttctca aagtgggttc attgcgaagt

1541                                                                    1610
gctgctctac tgtccctcct gctcgtcttc ccccagttct cgtttcgtct cttttttgtt cgttccatgc

1611                                                                    1680
actttctctc atactgtttt tgcctcttgt cgtacaagag gtgtatcaaa catgcagtac ctcgccgcgt

1681                                                                    1750
acgccctcgt ggcgctgtct ggcaagacgc cgtcgaaggc ggacgttcag gctgtcctga aggccgccgg

1751                                                                    1820
cgttgccgtg gatgcctccc gcgtggatgc cgtcttccag gaggtggagg gcaagagctt cgatgcgctg
                                          [L31
1821                                                                    1890
gtggccgagg gtcgcacgaa gctggtgggc tctggctctg ccgctcctgc tggcgctgtc tccactgctg

1891                                                                    1960
gtgccggcgc tggcgcggtg gccgaggcga agaaggagga gcccgaggag gaggaggccg atgatgacat

1961                                                                    2030
gggcttcggt ctctttgact aagcagcctc cgtgagtggt ctactgtcgt tactttttga cttgtttcat

2031                                                                    2100
ttgacttgtt ttcttcccgt aagcaaagaa cagagtaagc aagcatccat gcacgtcgaa gcgatgctac
                L31]
2101                                                                    2170
gaaccggtct ccctgctgcc catatcccct cagcgacggc gaccctccct cctttccacg tgctgacctc

2171                                                                    2240
atctcctcca atctttcacc ttctcttctg tctctcttct tgggttccct cttgaccgat cctgcatcga

2241                                                                    2310
ccatgaccag gtaattgcta tggctcttca agagaatagg gactcctaca aggtcaaagc tttttccttg

2311                                                                    2380
tccgagtttg tgcggcagtg ttccgactgc caccagctgt gcctcgagaa gacggcccgc gttgtggaaa

2381                                                                    2450
acagcagtcg tgccccgcgg ggatgggaag ccctattcaa ctccgttgcg gaggagcggc ggtctgagga
 
2451                                                                    2520
cttggacctc tgcgagtcgc gatgccggta catgcaggcc acctgcccgt cacaggagaa ggtccagcag

2521                                                                    2590
tacgagcagg ctctcgccgc cgcgttactc aagccgaaaa agagcacgcc cccaaacccg tctgtcttgc

2591                                                                    2660
aagacgcttc ccggtgcggc acgcggtcga ggttttctcg aacgtcctgt tgcccgtggc gcacaaaccc

2661                                                                    2730
agaaggtatc cttcccaacc aaagacctga aacgggaggc acaccgacgc cggtcccacg ccggacggcc

2731                                                                    2800
gacggaggcg accgaaggtg ctcgagggga ccaagacatg gacaacgata tttcgcgccg ctggagcaag

2801                                              2850
cttcgccaag agcgcttcgg ctcaaaggca gagcacgttg ctccgtcgac
SEQ ID NO:2 Sequence of nucleotides from the gene Lip2a that codes for the Lip2a protein and amino acid sequence of the Lip2a protein. 
SEQ.ID. NO.2

1                                                                                    60
ATG CAG TAC CTT GCC GCG TAC GCC CTC GTG GCG CTG TCT GGC AAG ACG CCG TCG AAG GCG
Met Gln Tyr Leu  Ala Ala Tyr Ala Leu Val Ala Leu Ser Gly Lys Thr Pro Ser Lys Ala
                 5                   10                  15                  20

61                                                                                   120
GAC GTT CAG GCT GTC CTG AAG GCC GCC GGC GTT GCC GTG GAT GCC TCC CGC GTG GAT GCC
Asp Val Gln Ala Val Lys Lys Ala Ala Gly Val Ala Val Asp Ala Ser Arg  Val Asp Ala
                 25                  30                  35                  40

121                                                                                  180
GTC TTC CAG GAG GTG GAG GGC AAG AGC TTC GAT GCG CTG GTG GCC GAG GGC CGC ACG AAG
Val Phe Gln Glu Val Glu Gly Lys Ser Phe Asp Ala Leu Val Ala  Glu Gly Arg Thr Lys
                 45                  50                  55                  60

181                                                                                  240
CTG GTG GGC TCT GGC TCT GCC GCT CCT GCT GGC GCT GTC TCC ACT GCT GGT GCC GGC GCT
Leu Val Gly Ser Gly Ser Ala Ala Pro Ala Gly  Ala Val Ser Thr Ala Gly Ala Gly Ala
                 65                  70                  75                  80

241                                                                                  300
GGC GCG GTG GCC GAG GCG AAG AAG GAG GAG CCC GAG GAG GAG GAG GCC GAT GAT GAC ATG
Gly Ala Val Ala Glu Ala Lys Lys Glu Glu Pro Glu Glu Glu Glu  Ala Asp Asp Asp Met
                 85                  90                  95                  100

301                     321
GGC TTC GGT CTC TTT GAC TAA
Gly Phe Gly Leu Phe Asp *
                    106
 
SEQ ID N° 3

1 50
MRGSHHHHHH TDPHASSVPS SLVIEGRISE FAAAFQREMQ YLAAYALVAL

51 100
SGKTPSKADV QAVLKAAGVA VDASRVDAVF QEVEGKSFDA LVAEGRTKLV

101 144
GSGSAAPAGA VSTAGAGAGA VAEAKKEEPE EEEADDDMGF GLFD
Amino acid sequence of the Lip2a protein (SEQ ID NO 2) expressed in the vector PQE-31 (SEQ. ID. NO. 3)

LiP2a      MQYLAAYALVALSGKT.PSKADVQAVLKAAGVAVDASRVDAVFQEVEGK 48
LiP2b    MSTKYLAAYALASLSKAS.PSQADVEAICKAVHIDVDQATLAFVMESVTGR 50
HuP2       MRYVASYLLAALGGNSSPSAKDIKKILDSVGIEADDDRLNKVISELNGK 49
TcP2       MKYLAAYALVGLSGGT.PSKSAVEAVLKAAGVPVDPRSVDALFAEFAGK 48

Lip2a    SFDALVAEGRTKL....VGSGSAAPAGAVSTAGAGAGAVAEAKK....E 89
                    |         |         |        | |
LiP2b    DVATLIAEGAAKMSAMPAASSGAAAGVTASAAGDAAPAAAAAKK....D 95
                    |         |         |        | |
HuP2     NIEDVIAQGIGKLASVPAGGAVAVSAAPGSAAPAAGSAPAAAEEKKDEK 98
                    |         |         |        | |
TcP2     DFDTVCTEGKSKLVGGVTRPNAATASAPTAAAAASSGAAAPAAA..... 92

Lip2a      EPEEEEADDDMGFGLFD 106
            ||  ||||||||||
LiP2b      EP.EEEADDDMGFGLFD 111
            ||  ||||||||||
HuP2       KEESEESDDDMGFGLFD 115
            ||  ||||||||||
TcP2       ..AEEEEDDDMGFGLFD 107

## Claims

1. A composition for stimulating an immune response in immunized individuals or in cell groups, **characterized in that** said composition consists of a saline solution that
- comprises Lip2a Leishmania protein represented by SEQ ID NO:2 or a polypeptide which differs there from only by conserved substitutions of the amino acids of SEQ ID NO:2 of which the amino acid groups that represent conserved changes are (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his, or
- comprises a nucleic acid encoding the Lip2a Leishmania protein as identified above, or being represented by SEQ ID NO:1.

2. A composition according to claim 1, **characterized in that** it is-further comprises an adjuvant such as biodegradable beads, ISS (i.e. immunostimulatory sequences), BCG, or liposomes.

3. A composition according to claim_1 or 2, **characterized in that** it further comprises at least one antigen.

4. A method for stimulating in vitro an immune response in mononuclear cells immunized or not with the protein Lip2a represented by SEQ ID NO:2, **characterized in that** it comprises incubating said mononuclear cells with the composition of claim 1.

5. A method according to claim 4, **characterized in that** said incubation further comprises at least one antigen.

6. A method according to claim 4 or 5, **characterized in that** said mononuclear cells are peripheral mononuclear cells.

7. Use of the composition as defined in any one of claims 1 to 3, for the manufacture of a medicament for stimulating an in vivo immune response against an antigen.

8. Use according to claim 7, **characterized in that** said composition is administered to an individual in the presence of at least one antigen.

9. Use of the composition as defined in claim 1, for the manufacture of an adjuvant in a vaccine for the prevention of Leishmaniasis.

10. Use of the composition as defined in claim 1, for the manufacture of a vaccine for the prevention of Leishmaniasis, **characterized in that** said composition is used as a subunit of said vaccine

## Patentansprüche

1. Zusammensetzung zur Stimulation einer Immunantwort in immunisierten Einzelpersonen oder in Zellgruppen, **dadurch gekennzeichnet, dass** die genannte Zusammensetzung aus einer Salin-Lösung besteht, die
- Lip2a Leishmania Protein umfasst, das durch SEQ ID NO:2 oder einem Polypeptid, das sich davon nur durch konservierte Substitutionen der Aminosäuren der SEQ ID NO:2 unterscheidet, dargestellt ist, wobei die Aminosäuregruppen, die konservierte Änderungen darstellen (1) Ala, Pro, Gly, Glu, Asp, Gln, Asn, Ser, Thr; (2) Cys, Ser, Tyr, Thr; (3) Val, Ile, Leu, Met, Ala, Phe; (4) Lys, Arg, His; und (5) Phe, Tyr, Trp, His, oder
- eine Nukleinsäure umfasst, die das wie oben identifizierte Lip2a Leishmania Protein verschlüsselt, oder durch SEQ ID NO:1 dargestellt ist.

2. Zusammensetzung gemäß Anspruch 1, **dadurch gekennzeichnet, dass** sie zusätzlich einen Hilfsstoff wie biologisch abbaubare Kugeln, ISS (z.B. immunostimulatorische Sequenzen), BCG oder Liposomen, umfasst.

3. Zusammensetzung gemäß Anspruch 1 oder 2, **dadurch gekennzeichnet, dass** sie zusätzlich zumindest ein Antigen umfasst.

4. Verfahren zur Stimulation einer in vitro Immunantwort in mononuklearen Zellen, die oder die nicht mit dem Lip2a Protein, welches durch SEQ ID NO:2 dargestellt ist, immunisiert sind, **dadurch gekennzeichnet, dass** es das Inkubieren der genannten mononuklearen Zellen mit der Zusammensetzung gemäß Anspruch 1 umfasst.

5. Verfahren gemäß Anspruch 4, **dadurch gekennzeichnet, dass** das genannte Inkubieren zusätzlich zumindest ein Antigen umfasst.

6. Verfahren gemäß Anspruch 4 oder 5, **dadurch gekennzeichnet, dass** die genannten mononuklearen Zellen periphere mononukleare Zellen sind.

7. Verwendung der Zusammensetzung gemäß einem der Ansprüche 1 bis 3 zur Herstellung eines Medikaments zum Stimulation einer in vivo Immunantwort gegen ein Antigen.

8. Verwendung gemäß Anspruch 7, **dadurch gekennzeichnet, dass** die genannte Zusammensetzung einer Einzelperson in der Gegenwart von zumindest einem Antigen verabreicht wird.

9. Verwendung der Zusammensetzung gemäß Anspruch 1 zur Herstellung eines Hilfsstoffes in einem Impfstoff zur Verhinderung von Leishmaniose.

10. Verwendung einer Zusammensetzung gemäß Anspruch 1 zur Herstellung eines Impfstoffs zur Verhinderung von Leishmaniose, **dadurch gekennzeichnet, dass** die genannte Zusammensetzung als eine Untereinheit des genannten Impfstoffes verwendet wird.

## Revendications

1. Une composition pour stimuler une réponse immunitaire chez des individus immunisés ou des groupes de cellules, **caractérisé en ce que** ladite composition consiste en une solution saline qui comprend:
- la protéine Leishmania Lip2a représentée par la séquence SEQ ID NO:2 ou un polypeptide qui n'en diffère que par les substitutions conservées des acides aminés de SEQ ID NO:2 dont les groupes d'acides aminés représentant les changements conservés sont: (1) ala, pro, gly, glu, asp, gln, asn, ser, thr; (2) cys, ser, tyr, thr; (3) val, ile, leu, met, ala, phe; (4) lys, arg, his; et (5) phe, tyr, trp, his
ou
- un acide nucléique codant pour la protéine Leishmania Lip2a telle qu'identifiée ci-dessus ou représenté par la séquence SEQ ID NO:1.

2. Une composition selon la revendication 1, **caractérisée en ce qu'**elle comprend en outre un adjuvant tels que des billes biodégradables, ISS (soit des séquences immunostimulantes), le BCG ou des liposomes.

3. Une composition selon la revendication 1 ou 2, **caractérisée en ce qu'**elle comprend en outre au moins un antigène.

4. Un procédé pour stimuler in vitro une réponse immunitaire dans des cellules mononucléaires immunisées ou non par la protéine Lip2a représentée par la séquence SEQ ID NO:2, **caractérisé en ce qu'**il comprend l'incubation desdites cellules mononucléaires par la composition de la revendication 1.

5. Un procédé selon la revendication 4, **caractérisée en ce que** ladite incubation comprend au moins un autre antigène.

6. Un procédé selon la revendication 4 ou 5, **caractérisé en ce que** lesdites cellules mononucléaires sont des cellules mononucléaires périphériques.

7. Utilisation de la composition telle que définie dans l'une quelconque des revendications 1 à 3, pour la fabrication d'un médicament pour stimuler une réponse immunitaire in vivo à l'encontre d'un antigène.

8. Utilisation selon la revendication 7, **caractérisée en ce que** ladite composition est administrée à un individu en présence d'au moins un antigène.

9. Utilisation de la composition telle que définie dans la revendication 1, pour la fabrication d'un adjuvant dans un vaccin pour la prévention de la Leishmaniose.

10. Utilisation de la composition telle que définie dans la revendication 1, pour la fabrication d'un vaccin pour la prévention de la Leishmaniose, **caractérisée en ce que** ladite composition est utilisée comme sous unité dudit vaccin.
